# EUROPÄISCHE PATENTSCHRIFT

(11) **EP 0 360 347 B1**
(45) Veröffentlichungstag und Bekanntmachung des Hinweises auf die Patenterteilung: **27.12.1995**
(21) Anmeldenummer: 89202353.2
(22) Anmeldetag: 18.09.1989
(51) Int. Cl.: G01N 23/201, A61B 6/02, G21K 1/02

(54) **Anordnung zur Messung des Impulsübertrags-Spektrums**
Arrangement for the measurement of the pulse transfer spectrum
Dispositif pour la mesure du spectre de transfert d'impulsions

(30) Priorität: 22.09.1988 DE 3832146; 21.03.1989 DE 3909147
(43) Veröffentlichungstag der Anmeldung: 28.03.1990
(73) Patentinhaber: Philips Patentverwaltung GmbH, 22335 Hamburg (DE); Philips Electronics N.V., 5621 BA Eindhoven (NL)
(72) Erfinder: Harding, Geoffrey, Dr., D-2000 Hamburg 53 (DE)
(74) Vertreter: Hartmann, Heinrich, Dipl.-Ing.

(56) Entgegenhaltungen:
- EP-A- 0 153 786
- DE-A- 2 003 753
- DE-A- 2 312 507

## Beschreibung

Die Erfindung betrifft eine Anordnung zum Messen des Impulsübertrags-Spektrums von in einem Untersuchungsbereich elastisch gestreuten Röntgenquanten, mit einem auf der einen Seite des Untersuchungsbereichs angeordneten polychromatischen Röntgenstrahler und einer auf der anderen Seite des Untersuchungsbereichs befindlichen, die Energie der Röntgenquanten messenden Detektoranordnung sowie mit zwei Blendenanordnungen mit gemeinsamer Symmetrieachse, die nur Streustrahlung innerhalb eines bestimmten Streuwinkelbereiches zum Detektor durchlassen, von denen die erste zwischen dem Untersuchungsbereich und dem Röntgenstrahler und die zweite zwischen dem Untersuchungsbereich und der Detektoranordnung angeordnet ist. Eine solche Anordnung ist im wesentlichen Gegenstand der deutschen Patentanmeldung P 37 12 928.

Bekanntlich besteht Streustrahlung, die mit der Richtung der Primärstrahlung nur einen kleinen Winkel einschließt (z.B. kleiner als 10°) überwiegend aus elastisch gestreuter Strahlung, wenn die Energie der Röntgenquanten nicht zu hoch ist. Im Gegensatz zu nicht elastisch gestreuter Strahlung (Compton-Streustrahlung) entspricht das Energiespektrum elastisch gestreuter Strahlung dem des Primärstrahlenbündels. Die Intensität elastisch gestreuter Strahlung weist eine starke Abhängigkeit vom Impulsübertrag auf, die von der molekularen Struktur des durchstrahlten Stoffes bestimmt wird.

Bei der Anordnung nach der deutschen Patentanmeldung P 37 12 928 wird diese auch für Fett und Muskelgewebe unterschiedliche Abhängigkeit dazu ausgenutzt, ein Fettbild bzw. ein Muskelgewebebild zu erstellen. Dabei wird die von einer monochromatischen Strahlenquelle erzeugte Streustrahlung für denjenigen Impulsübertragsbereich gemessen, bei dem das Spektrum des betreffenden Gewebes (Fett bzw. Muskel) möglichst groß und das der anderen Gewebeart (Muskel bzw. Fett) möglichst klein ist.

In der Anmeldung P 37 12 928 ist weiter angegeben, daß anstelle eines monochromatischen Strahlers auch ein polychromatischer Gamma- oder Röntgenstrahler vorgesehen sein kann, wenn energieauflösende Detektoren verwendet werden. Damit läßt sich das Impulsübertragsspektrum bestimmen.

Aus einem solchen Spektrum lassen sich Aufschlüsse über die molekulare Struktur des durchstrahlten Stoffes gewinnen, die der Identifizierung dieses Stoffes dienen können. Dies ist für medizinische Zwecke nützlich, z.B. zur Bestimmung des Mineralgehaltes von Knochen bei der Osteoporose-Diagnose - aber auch für andere Zwecke, beispielsweise zur Gepäckkontrolle.

Der Impulsübertrag eines Röntgenquants ist zumindest annähernd dem Produkt aus der Energie des Röntgenquants und dem Streuwinkel proportional, unter dem das Röntgenquant beim Streuprozeß aus seiner vorherigen Bahn abgelenkt wurde. Die Genauigkeit, mit der der Impulsübertrag bestimmt werden können, hängt daher davon ab, wie genau die Energie des Röntgenquants und sein Streuwinkel bestimmt werden kann. Die Energie von Röntgenquanten läßt sich mit Hilfe eines geeigneten Detektors, beispielsweise aus Germanium, recht genau messen. Die Genauigkeit, mit der sich der Streuwinkel bestimmen läßt, hängt bei der erwähnten Anordnung von den beiden Blendenanordnungen ab. Die erste Blendenanordnung ist so gestaltet, daß ein Primärstrahl mit geringem Querschnitt (pencil-beam) ausgeblendet wird. Die zweite Blendenanordnung besteht aus einer Anzahl von Lamellen, die auf den Mantelflächen von Kegeln angeordnet sind, deren Spitzen auf dem ausgeblendeten Primärstrahl im Untersuchungsbereich liegen. Wenn damit der Streuwinkel möglichst genau festgelegt werden soll, müssen die Lamellenabmessungen möglichst groß sein im Vergleich zur Dicke des Untersuchungsbereichs. Dies führt bei dicken Objekten zu sehr langen Lamellen und damit zu Detektoren mit sehr großem Durchmesser. Solche Detektoren sind teuer.

Aufgabe der vorliegenden Erfindung ist es, eine Anordnung der eingangs genannten Art so auszugestalten, daß auch bei dicken Objekten mit einer vergleichsweise kleinen Detektoranordnung eine relativ genaue Bestimmung des Impulsübertrages möglich ist, und zwar so, daß der Impulsübertrag gleichzeitig für verschiedene Tiefen des Untersuchungsbereichs bestimmt werden kann. Diese Aufgabe wird durch die im Hauptanspruch angegebenen Maßnahmen gelöst.

Bei der Erfindung wird also kein nadelförmiger Primärstrahl erzeugt, sondern ein Primärstrahlenkegel, d.h. ein Primärstrahlenbündel, das auf der Mantelfläche eines Kegelstumpfes liegt. Die zweite Blendenanordnung sorgt dafür, daß die einzelnen Detektoren der Detektoranordnung jeweils unter einem bestimmten Streuwinkel von Streustrahlung aus diesem Kegel getroffen werden, wobei jeder Detektor die Streustrahlung aus einem anderen Teil des Untersuchungsbereichs erfaßt. Aufgrund der Kegelform des Kegelstrahlenbündels ist es möglich, die zweite Blendenanordnung so auszugestalten, daß das von der Detektoranordnung registrierte Streustrahlenbündel zur Symmetrieachse hin konvergiert bzw. parallel dazu verläuft - und nicht divergiert, wie bei der bekannten Anordnung - , so daß auch bei einem großen Abstand zwischen Detektoranordnung und Untersuchungsbereich relativ kleine Detektoren verwendet werden können.

Es sei an dieser Stelle erwähnt, daß aus der DE-PS 20 03 753 bereits eine Anordnung zum Messen von Streukurven von (dünnen) Versuchskörpern bekannt ist, bei der die Röntgenstrahlung von einer Blendenanordnung so ausgeblendet wird, daß sich ein Primärstrahlenkegel ergibt. Die Streustrahlung wird von einem auf der Symmetrieachse des Primärstrahlenbündels angeordneten, nicht energieauflösenden Detektor (Zählrohr) durch eine ringförmige Blende hindurch erfaßt, die zusammen mit dem Detektor in Richtung der Symmetrieachse verschiebbar ist. Aus den dabei gewonnenen Meßwerten läßt sich die Streukurve des Versuchskörpers gewinnen, falls eine monochromatische Strahlung verwendet wird. Während also bei der bekannten Anordnung mit konstanter Energie und veränderlichem Streuwinkel gemessen wird, wird bei der Erfindung mit festem Streuwinkel bzw. fest angeordneten Blendenanordnungen in Abhängigkeit von der Energie der Röntgenquanten gemessen, was mit einem geeigneten Detektor relativ genau möglich ist.

Eine bevorzugte Weiterbildung der Erfindung sieht vor, daß die zweite Blendenanordnung aus mehreren einander umschließenden, zu der Symmetrieachse des Primärstrahlenkegels konzentrischen kegelmantelförmigen Kollimatorkörpern besteht. Wenn die Detektoranordnung dabei mehrere konzentrisch angeordnete, ringförmige Detektoren umfaßt, die jeweils die zwischen zwei Kollimatorkörpern hindurchtretende Streustrahlung erfassen, ist es möglich, gleichzeitig und mit hoher Effizienz die Streustrahlung in verschiedenen Tiefen des Untersuchungsbereichs zu messen.

Die Erfindung wird nachstehend anhand der Zeichnungen näher erläutert. Es zeigen:
Fig. 1 eine Anordnung zur Bestimmung des Impulsübertrages.
Fig. 2 eine zweite Ausführungsform
Fig. 3a und 3b eine für die Anordnung nach Fig. 2 geeignete Detektoranordnung,
Fig. 4a eine dritte nicht zur Erfindung gehörende Ausführungsform in einem die Symmetrieachse des Primärstrahlenkegels enthaltenden Querschnitt,
Fig. 4b einen dazu senkrechten Querschnitt durch die gleiche Anordnung und
Fig. 5 eine Variante der dritten Ausführungsform.

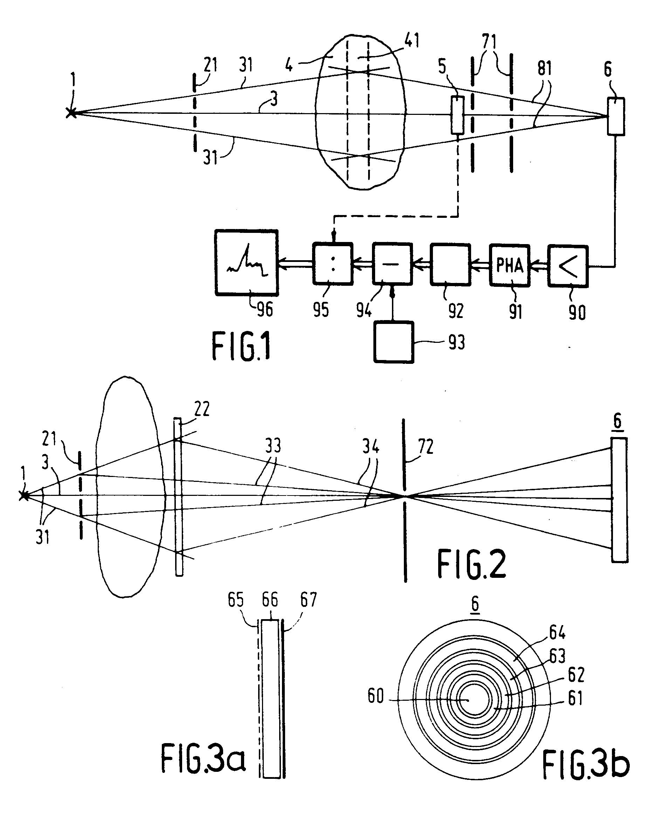

In Fig. 1 ist mit 1 ein Röntgenstrahler bezeichnet, dessen nicht näher dargestellte, vorzugsweise aus Wolfram bestehende Anode im Betriebszustand gegenüber der Kathode eine Spannung von z.B. 100 kV aufweist. Der überwiegende Teil der von einem solchen Röntgenstrahler emittierten Röntgenquanten besitzt eine Energie zwischen 40 und 80 keV. Zwischen dem Röntgenstrahler 1 und dem Untersuchungsbereich befindet sich eine erste Blendenanordnung, die aus einer Blendenplatte 21 mit einer kreisringförmigen Öffnung für den Durchtritt der vom Röntgenstrahler 1 emittierten Röntgenstrahlung besteht. Die durch die Öffnung der Blendenplatte 21 hindurchtretenden Röntgenstrahlen definieren somit die Mantelfläche eines Primärstrahlenkegels, dessen Spitze mit dem Fokus des Röntgenstrahlers 1 zusammenfällt und dessen Symmetrieachse durch das Zentrum der ringförmigen Öffnung verläuft. Die Blendenplatte 21 weist an dieser Stelle eine punktförmige Öffnung für den Durchtritt eines Primärstrahls 3 mit geringem Querschnitt auf. Dieser fällt somit mit der Symmetrieachse 3 des Primärstrahlenkegels 31 zusammen.

Der Primärstrahlenkegel erzeugt in dem Untersuchungsbereich 4 u.a. elastisch gestreute Strahlung. Von dieser Streustrahlung wird durch eine zwischen dem Untersuchungsbereich und einer Detektoranordnung 6 angeordneten zweite Blendenanordnung 71 diejenige Streustrahlung ausgeblendet, die im Primärstrahlenkegel 31 unter einem definierten kleinen Winkel von beispielsweise 3,6° gestreut wird. Die zweite Blendenanordnung 71 umfaßt zu diesem Zweck zwei Blendenplatten, die mit je einer zur Symmetrieachse 3 konzentrischen, ringförmigen Öffnung versehen sind. Der Durchmesser dieser Öffnung in der dem Untersuchungsbereich benachbarten Blendenplatte ist größer als der in der davon abgewandten Blendenplatte, so daß die Blende 71 Streustrahlung auf der Mantelfläche eine Sekundärstrahlenkegels 72 durchlassen kann, der sich zum Untersuchungsbereich hin öffnet. Der Schnittbereich der beiden Kegel 31 und 81, die die gleiche Symmetrieachse aufweisen, definiert eine zum Primärstrahl 3 senkrechte Schicht 41, aus der heraus Streustrahlung durch die Blende 71 hindurch auf die Detektoranordnung 6 treffen kann. Der Bereich innerhalb der Schicht, in dem diese Streustrahlung erzeugt wird, hat die Form eines zum Primärstrahl konzentrischen Ringes. Der Durchmesser dieses Ringes ergibt sich aus dem Öffnungswinkel des Primärstrahlenkegels bzw. des Sekundärstrahlenkegels, der jeweils 1,8° betragen kann. Die Abmessungen der Detektoranordnung 6 sind dabei unabhängig von der Dicke bzw. der Tiefe des Objektes 4, das mit dieser Anordnung untersucht werden soll.

Fig. 1 zeigt darüberhinaus ein Blockschaltbild der Auswerteeinrichtung, mit der aus dem Ausgangssignal der energieauflösenden Detektoranordnung, beispielsweise eines Germaniumdetektors, der Impulsübertrag des jeweils registrierten Röntgenquants bestimmt werden kann. Das Ausgangssignal des Detektors 6 wird über einen Verstärker 90 mit geeigneter Verstärkung einem Pulshöhenanalysator 91 zugeführt. Der Pulshöhenanalysator 91 ordnet jedem vom Detektor 6 erzeugten Ausgangsimpuls ein digitales Datenwort zu, das die Amplitude dieses Impulses und damit die Energie des Röntgenquants kennzeichnet. Zur Kennzeichnung des vom Detektor 6 zu registrierenden Energiebereiches können beispielsweise 128 verschiedene Datenworte vorgesehen sein. Eine Einheit 92 zählt, wie oft während einer vorgegebenen Meßzeit die einzelnen Datenworte auftreten und speichert diese Zahlen. Sie enthält somit am Ende der Meßzeit 128 Zahlen, die den Verlauf der Streuintensität als Funktion der Quantenenergie bzw. des Impulsübertrages kennzeichnen. Diese Zahlenfolge stellt somit das Energiespektrum bzw. das Spektrum des Impulsübertrages der elastisch gestreuten Strahlung dar. Dieses Spektrum muß jedoch noch korrigiert werden, um Störeinflüsse zu beseitigen.

Einer dieser Störeinflüsse besteht darin, daß die Röntgenstrahlung mit den Kanten der Blenden 21 und 71 in Wechselwirkung tritt, so daß der Detektor 6 selbst dann eine (Hintergrund-)Strahlung registriert, wenn das Objekt 4 im Untersuchungsbereich nicht vorhanden ist. Diese Hintergrundstrahlung ist vom Objekt 4 unabhängig. Sie kann daher einmal gemessen, in einem Speicher 93 gespeichert und in einer Schaltung 94 vom Ergebnis der Einheit 92 subtrahiert werden.

Ein weiterer Einfluß, der sich störend bemerkbar macht, besteht darin, daß in dem durch den Körper 4 transmittierten Spektrum die Intensität von der Energie der Röntgenquanten abhängt. Dies liegt zum einen daran, daß schon das von dem Röntgenstrahler 1 emittierte Spektrum eine derartige Abhängigkeit aufweist, und zum anderen daran, daß das Objekt 4 die verschiedenen Energien in unterschiedlicher Weise schwächt. Um diesen Einfluß auszugleichen, muß das vom Strahler 6 gemessene, von der Hintergrundstrahlung befreite Spektrum auf das transmittierte Spektrum normiert werden. Das transmittierte Spektrum kann aus dem Ausgangssignal des vor der Blende 71 im Primärstrahl angeordneten Detektors 5 abgeleitet werden - wie gestrichelt angedeutet. Die dafür erforderliche Elektronik - die Einheiten 90..92 - sind in Fig 1 der Übersicht halber nicht dargestellt. Die Normierung erfolgt in einer Schaltung 95, in der für jeden Teilbereich der Energie bzw. des Impulsübertrages die dafür mit dem Detektor 6 gemessene Intensität durch die transmittierte Intensität dividiert wird.

Das auf diese Weise korrigierte Energie- bzw. Impulsübertragsspektrum kann auf einer Wiedergabeeinheit 96 dargestellt werden. Für den Fall, daß lediglich untersucht werden soll, ob in dem Objekt 4, das beispielsweise ein Gepäckstück sein kann, ein vorbestimmter Stoff mit einem bekannten Impulsübertragsspektrum, beispielsweise Sprengstoff, enthalten ist, genügt es aber auch, wenn das gemessene Spektrum mit dem in einem weiteren Speicher enthaltenen Spektrum des gesuchten Stoffes verglichen wird und bei Übereinstimmung ein Alarmsignal erzeugt wird.

Die Funktion der Einheiten 92, 94 und 95 kann mit Hilfe eines Mikroprozessors realisiert werden, der darüberhinaus auch den gesamten Ablauf der Messung steuern kann.

Mit der in Fig. 1 dargestellten Anordnung kann immer nur ein bestimmter Volumenbereich des Objektes untersucht werden. Wenn das gesamte Objekt untersucht werden sollte, müßte entweder das Objekt oder die Meßanordnung in drei zueinander senkrechten Richtungen verschoben und jeweils eine Messung durchgeführt werden.

In Fig. 2 ist demgegenüber eine Anordnung dargestellt, die eine räumliche Auflösung in der Tiefe des Objektes, d.h. in Richtung des Primärstrahls, aufweist und bei der deshalb zur Untersuchung eines dreidimensionalen Objektes nur eine zweidimensionale Abtastung erforderlich ist. Diese Anordnung eignet sich daher in besondererem Maße für Gepäckkontrollen oder dergleichen.

Zwischen der Strahlenquelle 1 und dem Untersuchungsbereich befindet sich wiederum eine Blendenplatte 21 mit einer ringförmigen Öffnung zur Erzeugung eines Primärstrahlenkegels 31 und mit einer dazu konzentrischen Öffnung zur Erzeugung eines mit der Symmetrieachse des Kegels zusammenfallenden Primärstrahls 3. Wenn der Röntgenstrahler an einer Spannung von 150 kV betrieben wird und die Blendenplatte 21 von dem Strahler einen Abstand von 200 mm hat, sollte die ringförmige Öffnung zweckmäßigerweise einen Durchmesser von 14 mm aufweisen. Daraus ergibt sich ein Öffnungswinkel des Strahlenbündels von 2°.

Der Untersuchungsbereich wird durch eine für die Röntgenstrahlung transparente Platte 22 begrenzt, die von der Platte 21 einen Abstand von z.B. 250 mm hat. Mit der Anordnung nach Fig. 3 können also Objekte bis zu einer Dicke von 250 mm untersucht werden. In einem Abstand von der Blendenplatte 22, der deutlich größer ist als deren Abstand von dem Röntgenstrahler 1, beispielsweise 850 mm, befindet sich eine Lochblende 72, durch deren Öffnung die Symmetrieachse des Primärstrahlenkegels 31 verläuft. Die durch diese Öffnung hindurchtretende Strahlung trifft auf eine Detektoranordnung 6, die von der Lochblende 72 einen Abstand von z.B. 800 mm hat.

Wie sich aus Fig. 3a und 3b ergibt, umfaßt diese Detektoranordnung einen kreisförmigen Detektorkristall 66, der auf seiner einen Seite mit einer entsprechend großen kreisförmigen Elektrode 67 und auf seiner anderen Seite mit mehreren zueinander konzentrischen ringförmigen Elektroden versehen ist, die in ihrer Mitte eine kreisförmige Elektrode umschließen. Die Elektroden sind durch Zwischenräume von beispielsweise 0,5 mm voneinander getrennt. Die ringförmige Elektrode 65 mit dem größten Durchmesser führt Massepotential, ebenso wie die Elektrode 67. Diese wirkt für die anderen Elektroden daher als guard-elektrode. Die anderen Elektroden bilden zusammen mit dem Detektorkristall 66 und der gemeinsamen Elektrode 67 je einen Detektor 60..64, wobei der kreisförmige Detektor 60 in der Mitte von vier dazu konzentrischen ringförmigen Detektoren 61, 62, 63 und 64 umschlossen wird.

Der Durchmesser des kreisförmigen Detektors 60 ist so klein, daß er durch die Lochblende 72 hindurch nur von dem Primärstrahl getroffen werden kann, nicht aber von Streustrahlung, die der Primärstrahlenkegel 31 innerhalb des durch die Platten 21, 22 definierten Untersuchungsbereiches erzeugt. Der diesen Detektor 60 umschließende Ring 61 hat einen solchen Durchmesser, daß durch die Lochblende 72 hindurch von Streustrahlung getroffen werden kann, die in dem an die Blendenplatte 21 angrenzenden ersten Viertel des Untersuchungsbereiches durch den Primärstrahlenkegel 31 erzeugt wird. Ebenso erfassen der zweite, dritte und vierte Detektor 62, 63 und 64 die Streustrahlung, die der Primärstrahlenkegel im zweiten, dritten und vierten Viertel des Untersuchungsbereiches erzeugt.

Bei der Anordnung nach Fig. 2 muß jedem der Detektoren 61..64 die in Fig. 2 mit 90..96 bezeichnete Auswerteelektronik nachgeschaltet sein. Der zentrale Detektor 60 hat die gleiche Funktion wie der Detektor 5 bei den Anordnungen nach Fig. 1.

Die Randstrahlen des von der Detektoranordnung 6 registrierten Röntgenstrahlenbündels, die dem vorderen bzw. hinteren Rand des Untersuchungsbereiches zugeordnet sind, sind in der Zeichnung mit 33 und 34 bezeichnet. Man erkennt daraus, daß die dem letzten Viertel des Untersuchungsbereiches zugeordnete Streustrahlung den äußeren Detektor unter einem größeren Winkel erreicht als die Streustrahlung aus dem ersten Viertel des Untersuchungsbereichs den innersten Detektor. Die Unterschiede sind jedoch wesentlich kleiner, als sich aus der nicht maßstabsgerechten Darstellung in Fig. 2 ergibt; bei den angegebenen Abmessungen liegen die Streuwinkel zwischen 2,4 und 3,1°, so daß mit jedem der Detektoren 61..64 nahezu der gleiche Bereich des Impulsübertrages bestimmt werden kann. Dieser geringe Streuwinkelunterschied ist dadurch bedingt, daß der Abstand der Lochblende 72 von der Blende 22 wesentlich größer ist als deren Abstand von dem Röntgenstrahler 1. Dieser relativ große Abstand bewirkt auch, daß jeder Detektorring nur Streustrahlung empfängt, die einem sehr kleinen Streuwinkelbereich zugeordnet ist. Der äußerste Detktorring 64 beispielsweise empfängt Streustrahlung, die nur um 0,1° von einem mittleren Streuwinkel von ca. 2,95° abweicht. Bei den inneren Detektoren ist die Abweichung noch geringer.

Die Intensität der von der Detektoranordnung 6 gemessenen Streustrahlung ist umso größer und dementsprechend die Meßzeit umso kürzer, je größer die Öffnung in der Lochblende 72 ist. Eine größere Öffnung in der Lochblende hat aber zur Folge, daß jeder einzelne Detektor unter einem größeren Streuwinkelbereich von Streustrahlung getroffen werden kann, was die Genauigkeit beeinträchtigt, mit der der Impulsübertrag aus dem Detektorausgangssignal bestimmt werden kann. Der Durchmesser der Öffnung in der Lochblende ist daher ein Kompromiß zwischen der Meßzeit und der Genauigkeit, mit der der Impulsübertrag bestimmt werden kann. Ein für die zuvor genannten Abmessungen geeigneter Wert liegt bei etwa 3 mm.

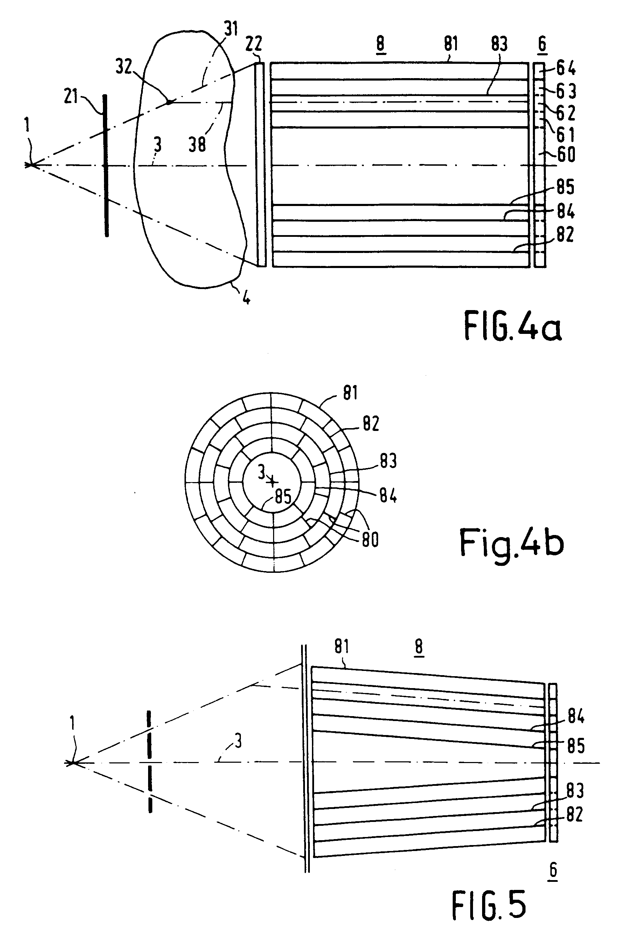

Bei der in den Fig. 4a und 4b, bzw. Fig. 5 dargestellten, bevorzugten Ausführungsform der Erfindung ist der Kompromiß zwischen der Meßzeit und der Genauigkeit, mit der der Impulsübertrag bestimmt werden kann, wesentlich günstiger als bei der Anordnung nach Fig. 2. Die Fig. 4a und 5 sind nicht maßstäblich; in Wirklichkeit sind die Abmessungen in horizontaler Richtung im Vergleich zu denen in vertikaler Richtung wesentlich größer.

Die von dem polychromatischen Röntgenstrahler 1 ausgehende Röntgenstrahlung fällt auf die Blendenplatte 21 mit einer Öffnung für den Primärstrahl 3 und einer dazu konzentrischen ringförmigen Öffnung, die den Primärstrahlenkegel 31 durchläßt, dessen Symmetrieachse mit dem Zentralstrahl 3 zusammenfällt. Der Öffnungswinkel des Primärstrahlenkegels, d.h. der Winkel zwischen einem von 1 ausgehenden Strahl dieses Kegels und dem Zentralstrahl 3 ist relativ klein, beispielsweise 3°. Der Untersuchungsbereich, in dem sich das Objekt 4 befindet, wird durch die Blendenplatte 21 und eine dazu parallele, für die Röntgenstrahlung transparente Platte 22 definiert. Der Abstand zwischen der Blende 21 und der Platte 22 kann 400 mm betragen, wobei der Abstand zwischen der Platte 22 und dem Röntgenstrahler 600 mm beträgt.

Unmittelbar an die Platte 22 schließt sich ein zylinderförmiger Kollimator 8 an, der eine Länge von beispielsweise 800 mm aufweist, und der konzentrisch zur Symmetrieachse 3 angeordnet ist. Dieser Kollimator wird durch eine Anzahl einander umschließender zylindermantelförmiger Kollimatorkörper 81..85 gebildet, die einen kreisförmigen Querschnitt aufweisen. In der Zeichnung sind nur fünf davon dargestellt, doch können mehr davon vorhanden sein, beispielsweise 8. Die erzielbare Auflösung in der Tiefe steigt mit der Zahl der Kollimatorkörper.

Der Innendurchmesser des äußersten Kollimatorkörpers 81 entspricht dem Durchmesser des Kreises, den der Primärstrahlenkegel 31 auf der Platte 22 beschreibt. Der Außendurchmesser des innersten Kollimatorkörpers 85 entspricht dem Durchmesser der ringförmigen Öffnung in der Blende 21 Die Kollimatorkörper, beispielsweise aus Stahlblech, sind so dünn wie möglich, doch dick genug, um die im Primärstrahlenkegel 31 erzeugte Streustrahlung zu absorbieren. Zwischen benachbarten Kollimatorkörpern ergibt sich jeweils die gleiche Durchmesserdifferenz.

Auf der von der Platte 22 abgewandten Öffnung des Kollimators 8 befindet sich die Detektoranordnung 6, die aus einer Anzahl von ringförmigen Detektoren 61..64 besteht, von denen jeder gerade die zwischen zwei benachbarten Kollimatorkörpern hindurchtretende Streustrahlung mißt. Der mittlere Detektor 60 mißt die Intensität der Röntgenstrahlung im Zentralstrahl 3. Die Signale werden in analoger Weise verarbeitet wie in Verbindung mit Fig. 1 im einzelnen erläutert.

In Fig. 4a ist mit 38 ein Streustrahl bezeichnet, der von einem Streupunkt 32 auf dem Primärstrahlenkegel 31 ausgeht und innerhalb des Untersuchungsobjektes 4 liegt. Dieser Streustrahl verläuft parallel zum Zentralstrahl 3 bzw. zur Symmetrieachse und entspricht daher einem Streuwinkel von 3°. Er tritt zwischen den Kollimatorkörpern 83 und 84 hindurch und trifft auf den Detektorring 62. Die Streustrahlung von Punkten, die dichter an der Platte 22 liegen, tritt durch weiter außen liegende Kollimatorkörper hindurch, während die Streustrahlung von Punkten in der Nähe der Blende 21 durch die weiter innen liegenden Kollimatorkörper hindurchtritt.

Man erkennt aus der Zeichnung, daß ein Streustrahl, der von dem gleichen Punkt ausgeht wie der Strahl 38 und der ebenfalls in der Zeichenebene liegt, nur wenig von der Richtung des Streustrahls 38 abweichen kann, z.B. 0,15°, weil er bei einer stärkeren Abweichung entweder vom Kollimatorkörper 84 oder vom Kollimatorkörper 83 absorbiert wird. Wenn hingegen der Streustrahl 38 um den Streupunkt 32 in tangentialer Richtung, d.h. aus der Zeichenebene heraus geschwenkt wird, können sich wesentlich größere Winkelabweichungen ergeben, ohne daß der Streustrahl von einem der Kollimatorkörper 83, 84 absorbiert wird. Trotzdem ist die dadurch bewirkte Änderung des Streuwinkels relativ klein: Wenn der Streustrahl 38 um den Streupunkt 32 in tangentialer Richtung um 0,5° geschwenkt wird, ergibt sich daraus eine Streuwinkeländerung von weniger als 0,05°. Jedem Streupunkt ist also ein relativ großer Bogen eines der ringförmigen Detektoren 61..64 zugeordnet, so daß der Detektor - bei gleichem Objekt 4, gleicher Intensität des Röntgenstrahlers 1 und gleicher Streuwinkelungenauigkeit - wesentlich mehr Streustrahlung empfängt als bei der Anordnung mit einer Lochblende nach Fig. 2.

Obwohl der Einfluß tangentialer Winkelabweichungen auf den Streuwinkel wesentlich geringer ist als der Einfluß radialer Winkelabweichungen, kann es zweckmäßig sein, auch die maximal möglichen tangentialen Winkelabweichungen zubegrenzen. Zu diesem Zweck sind gemäß Fig. 4b zwischen den Blendenkörpern 81..85 Lamellen 80 radial angeordnet, d.h. die Lamellen liegen in Ebenen, die sich in der Symmetrieachse 3 schneiden. Der Abstand zwischen zwei benachbarten Lamellen kann dabei aber mehrfach so groß sein wie der Abstandzwischen zwei benachbarten Kollimatorkörpern. Die Abstände zwischen benachbarten Lamellen, die jeweils zwischen den gleichen Kollimatorkörpern angeordnet sind, sind weitgehend unabhängig davon, ob die Lamellen sich zwischen den inneren oder den äußeren Kollimatorkörpern befinden. Infolgedessen sind zwischen den äußeren Kollimatorkörpern mehr Lamellen vorgesehen als zwischen den inneren - wie die Zeichnung zeigt.

Die kreis- bzw. ringförmigen Detektoren 60..64, die Halbleiterdetektoren aus hochreinem Germanium sein können, müssen den Abmessungen des Primärstrahlenkegels im Untersuchungsbereich entsprechen. Demnach müßte der ringförmige äußere Detektor 64 einen Durchmesser von rund 60 mm haben. Derartige Halbleiterdetektoren sind teuer. Das in Fig. 5 dargestellte Ausführungsbeispiel der Erfindung erlaubt die Verwendung von Detektorringen mit verringertem Durchmesser. Es unterscheidet sich von der Ausführungsform nach Fig. 4a lediglich dadurch, daß anstelle der zylindermantelförmigen Kollimatorkörper kegelmantelförmige Kollimatorkörper verwendet sind, die sich vom Untersuchungsbereich zum Detektor hin verjüngen, wobei die Kollimatorkörper so geformt sind, daß in einem den Zentralstrahl 3 enthaltenden Längsschnitt die Schnittgeraden der Kollimatorkörper parallel zueinander verlaufen. Beträgt der Öffnungswinkel der Kegel, auf deren Mantelflächen die Kollimatorkörper 81..85 liegen, z.B. 1°, dann kann der Durchmesser der Detektorringe bei sonst gleichen Abmessungen rund 28 mm kleiner sein als bei der Ausführungsform nach Fig. 4a.

Beim Ausführungsbeispiel nach Fig. 4 bzw. Fig. 5 ist jedem Detektor (z.B. 62) der Zwischenraum zwischen benachbarten Kollimatorkörpern (83,84) zugeordnet. Man kann die ringförmigen Detektoren aber auch breiter machen, so daß sie die Streustrahlung zwischen einem Kollimatorkörper (z.B. 83) und seinem übernächsten Nachbarn (85) erfassen. Dadurch wird zwar das Auflösungsvermögen in der Tiefe verringert, doch wird zugleich das Signal-Rausch-Verhältnis verbessert.

## Patentansprüche

1. Anordnung zum Messen des Impulsübertrags-Spektrums von in einem Untersuchungsbereich elastisch gestreuten Röntgenquanten, mit einem auf der einen Seite des Untersuchungsbereichs angeordneten polychromatischen Röntgenstrahler (1) und einer auf der anderen Seite des Untersuchungsbereichs (4) befindlichen, die Energie der Röntgenquanten messenden Detektoranordnung (6) sowie mit zwei Blendenanordnungen mit gemeinsamer Symmetrieachse (3), die nur Streustrahlung innerhalb eines bestimmten Streuwinkelbereiches zum Detektor durchlassen, von denen die erste zwischen dem Untersuchungsbereich und dem Röntgenstrahler und die zweite zwischen dem Untersuchungsbereich und der Detektoranordnung angeordnet ist,
dadurch gekennzeichnet, daß die erste Blendenanordnung (21) so gestaltet ist, daß die Röntgenstrahlung auf der Mantelfläche eines Primärstrahlenkegels (31) durchgelassen wird, daß die Detektoranordnung (6) mehrere konzentrisch angeordnete, ringförmige Detektoren (61 . . . 64) umfaßt, und daß die zweite Blendenanordnung so gestaltet ist, daß die erzeugte Streustrahlung rotationssymmetrisch zur Symmetrieachse auf der Mantelfläche eines Kegels (31; 32) auf die Detektoranordnung (6) trifft, und daß die einzelnen Detektoren (z.B. 62) von Streustrahlung (38) aus unterschiedlichen Tiefen (32) des Untersuchungsbereiches (4) mit einem festen Streuwinkel von weniger als 10° getroffen werden.

2. Anordnung nach Anspruch 1,
dadurch gekennzeichnet, daß die zweite Blendenanordnung (71) so gestaltet ist, daß sie einen Streustrahlenkegel 81 definiert, der sich vom Untersuchungsbereich zur Detektoranordnung (6) hin verjüngt.

3. Anordnung nach Anspruch 1,
dadurch gekennzeichnet, daß die zweite Blendenanordnung eine Lochblende (72) umfaßt, die im Abstand sowohl vom vom Untersuchungsbereich als auch von der Detektoranordnung (6) angeordnet ist und deren Öffnung mit der Symmetrieachse zusammenfällt.

4. Anordnung nach Anspruch 1,
dadurch gekennzeichnet, daß die zweite Blendenanordnung (8) aus mehreren einander umschließenden, zu der Symmetrieachse (3) des Primärstrahlenkegels (31) konzentrischen, kegelmantelförmigen Kollimatorkörpern (81..85) besteht.

5. Anordnung nach Anspruch 4,
dadurch gekennzeichnet, daß die Durchmesser der Kollimatorkörper und der Detektoren so aufeinander abgestimmt sind, daß die jeweils zwischen zwei Kollimatorkörpern hindurchtretende Strahlung von einem Detektor erfaßt wird.

6. Anordnung nach einem der vorhergehenden Ansprüche,
dadurch gekennzeichnet, daß die erste Blendenanordnung (21) in der Symmetrieachse eine Öffnung enthält, und daß auf der Symmetrieachse jenseits des Untersuchungsbereichs ein weiterer Detektor (5,60) zur Messung der transmittierten Strahlung vorgesehen ist.

7. Anordnung nach Anspruch 6,
dadurch gekennzeichnet, daß der weitere Detektor (5,60) die Streustrahlung energieaufgelöst mißt.

8. Anordnung nach Anspruch 6,
dadurch gekennzeichnet, daß der weitere Detektor (60) zusammen mit den ringförmigen Detektoren (61..64) einen gemeinsamen Detektorkristall umfaßt.

9. Anordnung nach einem der Ansprüche 3 oder 4,
dadurch gekennzeichnet, daß der Abstand der Detektoranordnung (6) vom Untersuchungsbereich (4) wesentlich größer ist als der Abstand des Röntgenstrahlers vom Untersuchungsbereich (4).

10. Anordnung nach Anspruch 4
dadurch gekennzeichnet, daß zwischen benachbarten Kollimatorkörpern Lamellen (80) in Ebenen angeordnet sind, die sich in der Symmetrieachse (3) schneiden.

## Claims

1. A device for measuring the pulse transfer spectrum of X-ray quanta which are elastically scattered in an examination zone, comprising a polychromatic X-ray source (1) which is arranged to one side of the examination zone and a detector device (6) which is situated to the other side of the examination zone (4) and which measures the energy of the X-ray quanta, and also comprising two diaphragm devices which have a common symmetry axis (3) and transmit scattered radiation to the detector only within a given scatter angle range, the first diaphragm device being arranged between the examination zone and the X-ray source whereas the second diaphragm device is arranged between the examination zone and the detector device, characterized in that the first diaphragm device (21) is constructed so that the X-rays on the envelope of a primary radiation cone (31) are transmitted that the detector device (6) comprises several concentrically arranged, annular detector (61 ... 64), that the second diaphragm device is constructed so that the scattered radiation produced is incident on the detector device (6) in a rotationally-symmetrical fashion with respect to the symmetry axis on the envelope of a cone (31;32), and that the individual detectors (for example, 62) are exposed to scattered radiation (38) from different depths (32) of the examination zone (4) with a fixed scatter angle of less than 10°.

2. A device as claimed in Claim 1, characterized in that the second diaphragm device (71) is constructed so that it defines a scattered radiation cone (81) which is tapered in the direction from the examination zone towards the detector device (6).

3. A device as claimed in Claim 1, characterized in that the second diaphragm device comprises a hole diaphragm (72) which is arranged at a distance from the examination zone as well as at a distance from the detector device (6) and whose aperture coincides with the symmetry axis.

4. A device as claimed in Claim 1, characterized in that the second diaphragm device (8) consists of a plurality of mutually enclosing conical collimator members (81 ... 85) which are concentric with the symmetry axis (3) of the primary radiation cone (31).

5. A device as claimed in Claim 4, characterized in that the diameters of the collimator members and the detectors are adapted to one another so that the radiation passing each time between two collimator members is detected by one detector.

6. A device as claimed in any one of the preceding Claims, characterized in that the first diaphragm device (21) comprises an aperture on the symmetry axis, and that on the symmetry axis on the other side of the examination zone there is provided a further detector (5, 60) for measuring the transmitted radiation.

7. A device as claimed in claim 6, characterized in that the further detector (5, 60) measures the scattered radiation in an energy-resolution fashion.

8. A device as claimed in Claim 6, characterized in that the further detector (60) and the annular detectors (61 ... 64) comprise a common detector crystal.

9. A device as claimed in one of the Claims 3 or 4, characterized in that the distance between the detector device (6) and the examination zone (4) is substantially larger than the distance between the X-ray source and the examination zone (4).

10. A device as claimed in Claim 4, characterized in that between neighbouring collimator members laminations (80) are arranged in planes which intersect at the symmetry axis (3).

## Revendications

1. Dispositif de mesure du spectre de transfert d'impulsions de quanta de rayons X dispersés de manière élastique dans une zone d'examen, comportant un générateur de rayons X polychromatiques (1) disposé d'un côté de la zone d'examen (4) et un dispositif de détection (6) disposé de l'autre côté de la zone d'examen et mesurant l'énergie des quanta de rayons X, ainsi que deux dispositifs à diaphragme ayant un axe de symétrie commun (3), qui ne laissent passer vers le détecteur que le rayonnement diffusé qui s'inscrit dans un domaine d'angle de diffusion déterminé, le premier étant disposé entre la zone d'examen et le générateur de rayons X et le deuxième, entre la zone d'examen et le dispositif de détection, caractérisé en ce que le premier dispositif à diaphragme (21) est conçu de manière à laisser passer les rayons X suivant l'enveloppe d'un cône de rayonnement principal (31), que ledit dispositif de détection (6) comprend plusieurs détecteurs annulaires (61 ... 64) disposés concentriquement, que le deuxième dispositif à diaphragme est conçu, de telle sorte que le rayonnement diffusé produit arrive sur le dispositif de détection (6) avec une symétrie de révolution par rapport à l'axe de symétrie suivant l'enveloppe d'un cône (31; 32), et que les détecteurs individuels (par exemple 62) sont frappés par le rayonnement diffusé (38) provenant de diverses profondeurs (32) de la zone d'examen (4) sous un angle de diffusion fixe inférieur à 10°.

2. Dispositif selon la revendication 1, caractérisé en ce que le deuxième dispositif à diaphragme (71) est conçu, de telle sorte qu'il définisse un cône de rayonnement diffusé (81) qui va en s'amincissant de la zone d'examen vers le dispositif de détection (6).

3. Dispositif selon la revendication 1, caractérisé en ce que le deuxième dispositif à diaphragme comprend un diaphragme perforé (72) qui est disposé à distance autant de la zone d'examen que du dispositif de détection (6) et dont l'ouverture coïncide avec l'axe de symétrie.

4. Dispositif selon la revendication 1, caractérisé en ce que le deuxième dispositif à diaphragme (8) est constitué de plusieurs corps collimateurs (81 ... 85) à enveloppe conique concentriques par rapport à l'axe de symétrie (3) du cône de rayonnement principal (31) et s'entourant les uns les autres.

5. Dispositif selon la revendication 4, caractérisé en ce que les diamètres des corps collimateurs et des détecteurs sont accordés l'un sur l'autre, de telle sorte que le rayonnement passant respectivement à travers deux corps collimateurs soit capté par un détecteur.

6. Dispositif selon l'une quelconque des revendications précédentes, caractérisé en ce que le premier dispositif à diaphragme (21) contient une ouverture dans l'axe de symétrie et en ce qu'il est prévu sur l'axe de symétrie, de chaque côté de la zone d'examen, un autre détecteur (5, 60) pour mesurer le rayonnement transmis.

7. Dispositif selon la revendication 6, caractérisé en ce que l'autre détecteur (5, 60) mesure le rayonnement diffusé sur la base de la dissipation d'énergie.

8. Dispositif selon la revendication 6, caractérisé en ce que l'autre détecteur (60) contient, conjointement avec les détecteurs annulaires (61 ... 64), un cristal de détection commun.

9. Dispositif selon l'une ou l'autre des revendications 3 et 4, caractérisé en ce que la distance du dispositif de détection (6) à la zone d'examen (4) est sensiblement plus grande que la distance du générateur de rayons X à la zone d'examen (4).

10. Dispositif selon la revendication 4, caractérisé en ce qu'entre des corps collimateurs voisins, des lamelles (80) sont disposées dans des plans qui se coupent sur l'axe de symétrie (3).
